# EUROPEAN PATENT SPECIFICATION

(11) **EP 0 609 301 B1**
(45) Date of publication and mention of the grant of the patent: **17.01.1996**
(21) Application number: 92921572.1
(22) Date of filing: 20.10.1992
(51) Int. Cl.: A61K 7/48

(54) **TOILETRIES COMPOSITION**
KÖRPERPFLEGEMITTEL
COMPOSITION DE TOILETTE

(30) Priority: 25.10.1991 GB 9122674
(43) Date of publication of application: 10.08.1994
(73) Proprietor: The Boots Company PLC, Nottingham Nottinghamshire NG2 3AA (GB)
(72) Inventor: HINKS, Jane Elizabeth The Boots Company plc, Nottingham NG2 3AA (GB)
(74) Representative: Kirk, Martin John
(86) International application number: EP9202423
(87) International publication number: WO9307855

(56) References cited:
- EP-A- 0 327 382
- FR-A- 2 292 027
- GB-A- 2 091 553
- US-A- 5 139 782
- PATENT ABSTRACTS OF JAPAN vol. 6, no. 231 (C-135)
- PATENT ABSTRACTS OF JAPAN vol. 10, no. 230 (C-365)(2286)
- CHEMICAL ABSTRACTS, vol. 88, no. 16, 17 April 1978, Columbus, Ohio, US; abstract no. 110388g, ITO 'surfactant free cosmetics'

## Description

The present invention relates to skin cleansing compositions and, in particular, to make-up removing compositions for use in cleansing sensitive skin such as the area around the eyes.

The term "skin cleansing" as used herein includes the cleansing of, and in particular the removal of make-up from, the eye-brows and eye-lashes.

Prior art skin cleansing compositions can be described variously as oil-based, emulsion-type or water-based detergent systems.

Oil-based skin cleansing compositions typically consist of a mixture of oils, normally mineral oils, and esters. These compositions can be irritant, particularly when used on sensitive skin, because they may contain surfactants and because they tend to leave a greasy film over the skin after use. This is particularly troublesome when cleaning around the eye, because the residual greasy film may run into the eye causing occular irritation.

Emulsion-type skin cleansing compositions are usually based on a class of surfactants known as emulsifiers, or on products of the reaction between organic acids and suitable bases to form salts, often referred to as "soaps", and can also contain further surfactants. Any such soap and surfactant species can be irritant, especially when used around the eye.

Oil-in-water emulsions are the more common, but water-in-oil emulsions are also available. In either case, however, emulsion-type preparations contain oils and/or waxes and tend to leave a greasy film on the skin as described above, with corresponding risks of ocular irritation.

Water-based detergent systems normally contain a surfactant as the main cleansing agent. This can cause irritation to the skin, particularly when the compositions are used in the area of the eyes, since there is the possibility that the surfactant may run into the eye during or after use.

The present invention seeks to provide a skin cleansing composition suitable for use on sensitive areas of skin and on eye-brows and eye-lashes which avoids these disadvantages but which is nonetheless effective to remove contaminants.

Patent Astracts of Japan, Vol.10, No.230, (C-365) (2286), 9/8/86, discloses a sebum-controlling cosmetic containing inter alia 0.05 to 3 weight % of a clay mineral and 25 to 40 weight % of a lower alcohol, preferably ethanol.

Hectorite-type clays, such as those available commercially from Laporte Industries under the trade names LAPONITE® XLG and LAPONITE® XLS, are already known in the art as gelling and thickening agents. Laponite® gels are marketed in the form of a granular free-flowing powder. When the powder is dispersed in water it forms a thixotropic gel. The manufacturer's formulary describes the use of Laponite® hectorite-type clays as thickening agents in inter alia shampoos, after-shave preparations and face masks. However, all these formulations are either oily, contain surfactants, or contain hectorite-type clay in amounts which would cause irritation to sensitive skin such as that found around the eyes.

It has been noted that hectorite-type clays have the ability to absorb dirt and oils when present at high levels. However, the levels described are unacceptable in a preparation for use on sensitive skin, since the clays are alkaline and therefore irritant when present at such levels.

It has now surprisingly been found that hectorite-type clays will act as cleansers in non-oily skin cleansing compositions at unexpectedly low levels. At the low clay levels of the present invention the pH of the composition may be kept tolerably low, typically less than about pH 8.5, without adjustment. As a result the compositions of the present invention are exceptionally gentle and non-irritant in use.

The term "hectorite-type clay" as used herein includes both naturally occurring hectorite clays and synthetic hectorite clays such as those available commercially under the trade names LAPONITE® XLG and LAPONITE® XLS. The chemical compositions of these latter clays approximate to magnesium lithium silicate and magnesium sodium silicate respectively.

Surprisingly, the cleansing action of the hectorite-type clay is such that substantially no surfactants are necessary to make up an effective cleansing composition. This is in direct contrast to the prior art non-oily skin cleansing compositions which almost invariably contain a surfactant in some form.

The present invention provides a non-oily skin cleansing composition comprising a skin-cleansing amount of a hectorite-type clay up to about 2% by weight of the composition and a preservative, said composition being substantially free of surfactants.

Preferably, the hectorite-type clay is present in an amount between about 0.25% and 1.8% by weight, suitably about 1.6% by weight of the composition.

The compositions of the present invention, being substantially free of surfactants, show exceptional gentleness, and are ideally suited to the cleansing of sensitive skin, such as that found in the region of the eyes. The compositions of the invention are well suited to cleaning heavily soiled sensitive skin and are therefore particularly effective as eye make-up removers. They are also ideally suited to removal of make-up from the eye-brows and eye-lashes.

The compositions of the invention have the cosmetic advantage that they may, if desired, be prepared in a highly clear or so-called "crystal" clear form. However, they may also be rendered "cloudy" by use of suitable additives, such as pigments, if preferred.

Suitably, the clay is a synthetic hectorite-type clay such as the clays available commercially under the trade names LAPONITE® XLG and LAPONITE® XLS. Such clays are readily available in large quantities. However, it is expected that naturally occurring hectorite clays of sufficient purity could also be used with satisfactory results.

In an embodiment, the composition further comprises a cellulose gum such as the gum available commercially from Hercules Chemical under the trade name CMC 7HF HERCULES®. This combination of hectorite-type clay and cellulose gem shows surprising synergistic thickening properties, so that the overall levels of thickener/gellant can be reduced, and the pH of the system thereby controlled. Use of this combination also gives rise to a surprising increase in gel stability.

Preferably, the composition has a pH below about 8.5, preferably between about 8.0 and 8.3 and suitably about 8.15. A pH above 8.5 is more likely to cause skin irritation to the user, and should therefore be avoided.

Suitably, the preservative may be methylparaben, butylparaben, ethylparaben or propylparaben, or preferably the blend of all four available commercially from Nipa Laboratories under the trade name NIPASTAT.

The compositions may if desired include a compound such as 1,3-butylene glycol to improve skin feel, or a dye such as FD & C Yellow No. 6 A1 Lake to improve appearance. Further excipients are well known in the formulator's art. However, the presence or absence of such optional components does not normally affect the action of the composition as a skin cleanser.

In a further aspect of the invention, there is provided a skin cleansing composition comprising the combination of a hectorite-type clay and a cellulose gem, As discussed above, this particular combination displays surprising synergistic properties which enable the overall levels of thickener/gellant to be reduced. The hectorite-type clay and cellulose gum are present individually at levels such that in combination they provide an effective skin cleanser.

Suitably the cellulose gum is the gum available commercially from Hercules Chemical under the trade name CMC 7HF Hercules®. Preferably the gum is present in an amount of between about 0.1% and 5% by weight of the composition, suitably between about 0.2% and 0.8% by weight. It is desirable that the composition comprises at least about 0.2% by weight of the gum since below this level the compositions are slightly less stable at higher temperatures. Preferably the gum is present in an amount of about 0.4% by weight.

In a further aspect of the invention, there is provided a method of cleansing the skin by application of a non-oily composition comprising a skin cleansing amount of a hectorite-type clay up to about 2% by weight of the composition, said composition being substantially free of surfactants. This method of cleansing is especially suited to the cleansing of sensitive skin such as the skin around the eyes, and the method finds particular application in the removal of eye make-up. The method of the present invention is also ideally suited to the removal of make-up from the eye-brows and eye-lashes, where exceptional gentleness of action is required to avoid ocular irritation.

Preferably, the clay is a synthetic clay, such as those available under the trade names Laponite® XLG and Laponite® XLS.

Suitably, the composition has a pH below about 8.5, preferably between about 8 and 8.3.

Preferably, the clay is present in an amount of between about 0.25% and 1.8% by weight of the composition.

In an embodiment, the composition further comprises a cellulose gum in an amount sufficient to exhibit synergism with the clay. Preferably the cellulose gum is the gum available commercially under the trade name CMC 7HF Hercules®. Beneficially, the gum is present in an amount between about 0.1% and 5% by weight of the composition, suitably between about 0.2% and 0.8% and preferably about 0.4%.

In a further aspect there is provided the use of a non-oily composition comprising up to about 2% by weight of a hectorite-type clay to assist in the removal of impurities from the skin, especially in the removal of make-up from sensitive skin such as that found around the eyes.

In a still further aspect of the invention, there is provided a method of preparing a composition as defined above by combining a skin cleansing amount of a hectorite-type clay with water, a preservative, and, optionally, any excipients desired, such that the clay is present up to about 2% by weight of the composition.

The invention will now be illustrated by the following Examples.

### Example 1

A skin cleansing composition comprising the following components was prepared:

| Component | % w/w |
|---|---|
| Synthetic hectorite-type clay (sold under the trade name Laponite® XLG) | 1.63 |
| Cellulose gum (sold under the trade name CMC 7HF Hercules®) | 0.43 |
| 1,3-butylene glycol | 5.42 |
| Methylparaben/butylparaben/ethylparaben/propylparaben blend (sold under the trade name Nipastat) | 0.02 |
| FD & C Yellow No.6 Al Lake (1% aqueous solution) | 0.00016 |
| Purified water | to 100 |

### Method

The hectorite-type clay was dispersed in the purified water by use of a high shear mixer/homogeniser of the type available under the trade name Silverson far about 10 to 15 minutes. The cellulose gum was then added and dispersed throughout the mixture by use of the Silverson Mixer.

The paraben blend was dissolved in the 1,3-butylene glycol by gentle warming and the FD & C Yellow No. 6 A1 Lake solution added.

The clay/cellulose gum solution was heated to 80°C with stirring by spatula until the gel had set up and cleared.

The paraben blend/butylene glycol/A1 Lake solution was added to the heated clay/cellulose gum solution and the product was stirred with a spatula until uniform.

The product was cooled to room temperature, made up to weight with water, and stirred with a spatula until uniform.

The pH of the product was 8.15. The gel was clear, firm and non-tacky. The viscosity could not be accurately measured since the product was highly aerated.

The product was found to be effective in removing make-up from the region of the eyes and from the eye-lashes without causing skin or occular irritation.

### Examples 2 to 6

Further skin cleansing compositions were prepared as shown in Table 1. In each case, the method of preparation was substantially as described under Example 1 but with variation in the concentration of hectorite-type clay as shown. Each gel was found to be effective in removing make-up from the region of the eyes and from the eye-lashes without causing skin or occular irritation.

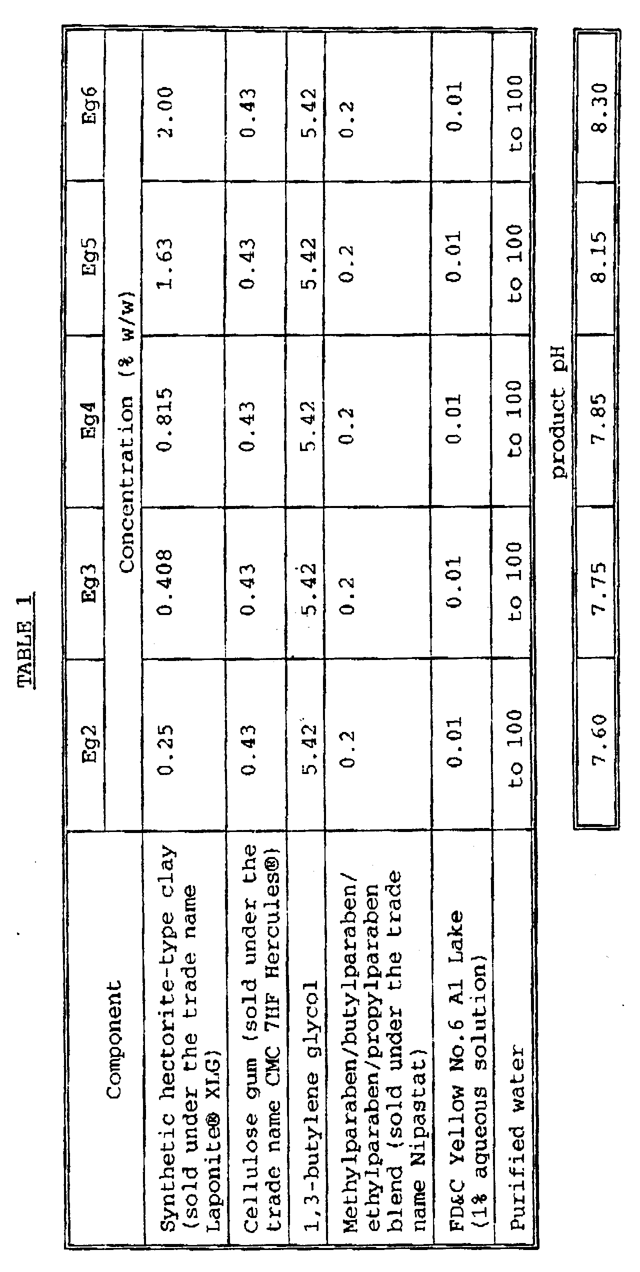

### Example A (Comparative)

A skin cleansing composition was prepared as shown below. The method of preparation was substantially as described under Example 1 except that the hectorite-type clay was omitted.

| Component | % w/w |
|---|---|
| Cellulose gum (sold under the trade name CMC 7HF Hercules®) | 0.43 |
| 1,3-butylene glycol | 5.42 |
| Methylparaben/butylparaben/ethylparaben/propylparaben blend (sold under the trade name Nipastat) | 0.2 |
| FD & C Yellow No.6 A1 Lake (1% aqueous solution) | 0.01 |
| Purified water | to 100 |

The product (pH 6.50) was thin and runny and was ineffective as a cleansing composition.

### Examples 7 to 12

Further skin cleansing compositions were prepared as shown in Table 2. In each case the method of preparation was substantially as described under Example 1 wish variation in the concentration of cellulose gum as shown. Each gel was found to be effective in removing make-up from the region of the eyes and from the eye-lashes without causing skin or ocular irritation.

It was found that those compositions comprising less than 0.2% cellulose gum were slightly less stable at elevated temperatures. In general it was found that the higher the concentration of cellulose gum, the thicker was the gel.

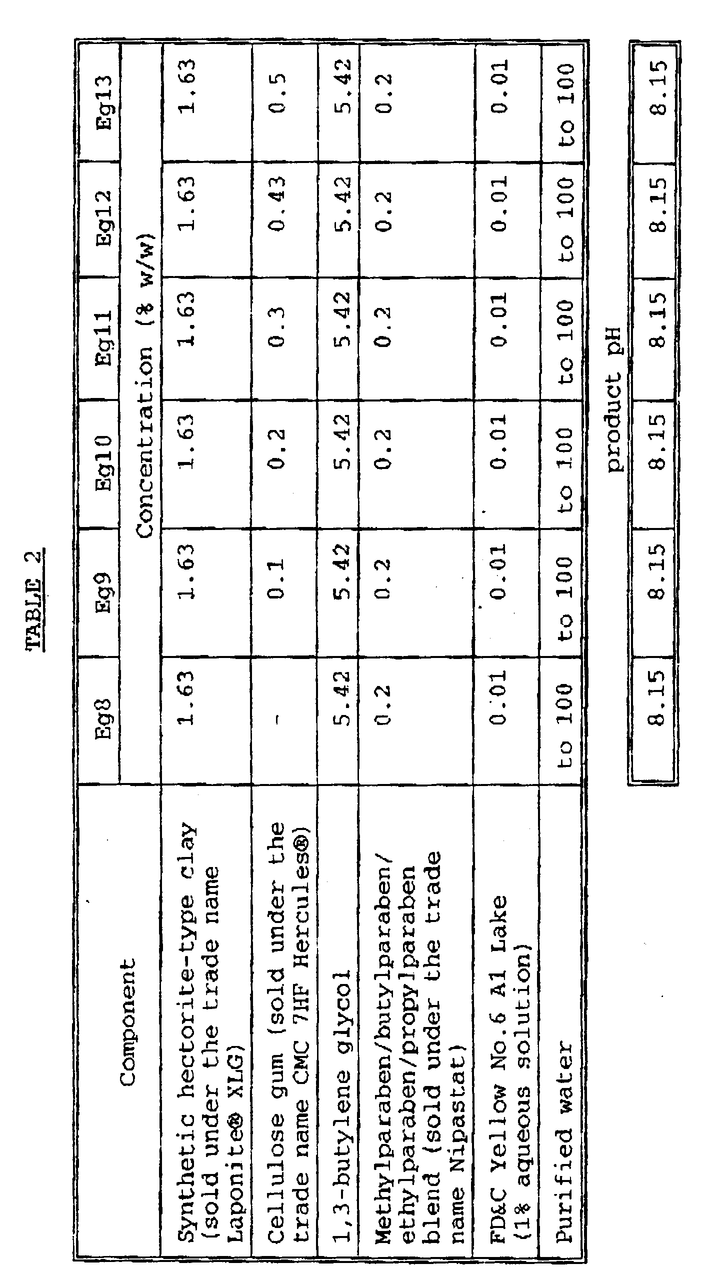

## Claims (Claims for the following Contracting State(s): AT, BE, CH, LI, DE, DK, FR, GB, GR, IE, IT, LU, MC, NL, SE)

1. A non-oily skin cleansing composition comprising a skin-cleansing amount of a hectorite-type clay in an amount of up to about 2% by weight of the composition and a preservative, said composition being substantially free of both surfactants and ocular irritants.

2. A composition as claimed in Claim 1 therein the hectorite-type clay is present in an amount of between about 0.25% and 1.8% by weight.

3. A composition as claimed in Claim 1 or Claim 2 wherein the hectorite-type clay is a synthetic clay.

4. A composition as claimed in any one of the preceding claims with a pH below about 8.5.

5. A composition as claimed in any one of the preceding claims comprising a cellulose gum as a thickener.

6. A composition as claimed in Claim 5 wherein the cellulose gum is a carboxymethylcellulose gum.

7. A composition comprising a hectorite-type clay and a cellulose gum in amounts sufficient to exhibit a synergistic increase in viscosity and a preservative, said composition being substantially free of both surfactants and ocular irritants.

8. A non-oily skin cleansing composition consisting essentially of a hectorite-type clay in an amount of up to about 2% by weight and a preservative and, optionally, a thickener, a colouring agent and a humectant, and being substantially free of both surfactants and ocular irritants.

9. A composition as claimed in Claim 7 or Claim 8 wherein the hectorite-type clay and/or cellulose gum are included as defined in any one of claims 1 to 6.

10. A method of cleansing the sensitive skin found in the region of the eyes by application of a non-oily composition comprising a skin-cleansing amount of a hectorite-type clay up to about 2% by weight, said composition being substantially free of both surfactants and ocular irritants.

11. A method as claimed in Claim 10 wherein the composition is as defined in any one of claims 1 to 9.

12. The use of a hectorite-type clay in an amount of up to about 2% by weight in a skin cleansing composition substantially free of both surfactants and ocular irritants to assist in the removal of impurities from sensitive skin or hair.

13. A method of preparing a composition as claimed in anyone of claims 1 to 9 by combining a hectorite-type clay with a preservative and a suitable solvent.

## Claims (Claims for the following Contracting State(s): ES)

1. A method of preparing a non-oily skin cleansing composition comprising combining a skin-cleansing amount of a hectorite-type clay in an amount of up to about 2% by weight of the composition with water and a preservative, said composition being substantially free of both surfactants and ocular irritants.

2. A method as claimed in Claim 1 wherein the hectorite-type clay is present in an amount of between about 0.25% and 1.8% by weight.

3. A method as claimed in Claim 1 or Claim 2 wherein the hectorite-type clay is a synthetic clay.

4. A method as claimed in any one of the preceding claims in which the composition prepared has a pH below about 8.5.

5. A method as claimed in any one of the preceding claims comprising addition of a cellulose gum as a thickener.

6. A method as claimed in Claim 5 wherein the cellulose gum is a carboxymethylcellulose gum.

7. A method of preparing a composition comprising combining a hectorite-type clay, with a cellulose gum, in amounts sufficient to exhibit a synergistic increase in viscosity, and a preservative, said composition being substantially free of both surfactants and ocular irritants.

8. A method of preparing a non-oily skin cleansing composition consisting essentially of combining a hectorite-type clay in an amount of up to about 2% by weight with a preservative and, optionally, a thickener, a colouring agent and a humectant, said composition being substantially free of both surfactants and ocular irritants.

9. A method as claimed in Claim 7 or Claim 8 wherein the hectorite-type clay and/or cellulose gum are included as defined in any one of Claims 1 to 6.

10. A method of cleansing the sensitive skin found in the region of the eyes by application of a non-oily composition comprising a skin-cleansing amount of a hectorite-type clay up to about 2% by weight, said composition being substantially free of both surfactants and ocular irritants.

11. A method as claimed in Claim 10 wherein the composition is as defined in any one of Claims 1 to 9.

12. The use of a hectorite-type clay in an amount of up to about 2% by weight in a skin cleansing composition substantially free of both surfactants and ocular irritants to assist in the removal of impurities from sensitive skin or hair.

13. A composition preparable by any of the methods of Claims 1 to 9.

## Patentansprüche (Patentansprüche für folgende(n) Vertragsstaat(en): AT, BE, CH, LI, DE, DK, FR, GB, GR, IE, IT, LU, MC, NL, SE)

1. Nichtölige Zubereitung zum Reinigen der Haut, enthaltend eine die Haut reinigende Menge Ton vom Hectorit-Typ von bis zu etwa 2 Gew.-% der Zubereitung und ein Konservierungsmittel, wobei die Zubereitung im wesentlichen frei von sowohl oberflächenaktiven als auch die Augen reizenden Mitteln ist.

2. Zubereitung gemäß Anspruch 1, in der der Ton vom Hectorit-Typ in einer Menge zwischen etwa 0,25 und 1,8 Gew.-% vorliegt.

3. Zubereitung gemäß Anspruch 1 oder 2, in der der Ton vom Hectorit-Typ ein synthetischer Ton ist.

4. Zubereitung gemäß einem der vorstehenden Ansprüche mit einem pH unterhalb von etwa 8,5.

5. Zubereitung gemäß einem der vorstehenden Ansprüche, welche Cellulosegummi als Verdickungsmittel enthält.

6. Zubereitung gemäß Anspruch 5, in der das Cellulosegummi Carboxymethylcellulosegummi ist.

7. Zubereitung, die einen Ton vom Hectorit-Typ und Cellulosegummi in ausreichender Menge, um eine synergistische Erhöhung der Viskosität zu zeigen, sowie ein Konservierungsmittel enthält, wobei die Zubereitung im wesentlichen frei von oberflächenaktiven und die Augen reizenden Mitteln ist.

8. Nichtölige Zubereitung zum Reinigen der Haut, die im wesentlichen aus Ton vom Hectorit-Typ in einer Menge von bis zu etwa 2 Gew.-%, einem Konservierungsmittel und ggf. einem Verdickungsmittel, einem Farbstoff und einem Feuchthaltemittel besteht und im wesentlichen frei von sowohl oberflächenaktiven als auch die Augen reizenden Mitteln ist.

9. Zubereitung gemäß Anspruch 7 oder in der der Ton vom Hectorit-Typ und/oder das Cellulosegummi gemäß einem der Ansprüche 1 bis 6 enthalten sind.

10. Verfahren zum Reinigen der im Bereich der Augen befindlichen, empfindlichen Haut durch Auftragen einer nichtöligen Zubereitung, die eine die Haut reinigende Menge Ton vom Hectorit-Typ von bis zu etwa 2 Gew.-% enthält, wobei die Zubereitung im wesentlichen frei von oberflächenaktiven und die Augen reizenden Mitteln ist.

11. Verfahren gemäß Anspruch 10, bei dem die Zubereitung gemäß einem der Ansprüche 1 bis 9 definiert ist.

12. Verwendung von Ton vom Hectorit-Typ in einer Menge von bis zu etwa 2 Gew.-% in einer Zubereitung zum Reinigen der Haut, die im wesentlichen frei von oberflächenaktiven und die Augen reizenden Mitteln ist, zum Unterstützen der Entfernung von Verunreinigungen von empfindlicher Haut oder Haaren.

13. Verfahren zur Herstellung gemäß einem der Ansprüche 1 bis 9, in dem man einen Ton vom Hectorit-Typ mit einem Konservierungsmittel und einem geeigneten Lösungsmittel vereinigt.

## Patentansprüche (Patentansprüche für folgende(n) Vertragsstaat(en): ES)

1. Verfahren zur Herstellung einer nichtöligen Zubereitung zum Reinigen der Haut, bei dem man eine die Haut reinigende Menge Ton vom Hectorit-Typ von bis zu etwa 2 Gew -% der Zubereitung mit Wasser und einem Konservierungsmittel kombiniert, wobei die Zubereitung im wesentlichen frei von sowohl oberflächenaktiven als auch die Augen reizenden Mitteln ist.

2. Verfahren gemäß Anspruch 1, wobei der Ton vom Hectorit-Typ in einer Menge zwischen etwa 0,25 und 1,8 Gew.-% vorliegt.

3. Verfahren gemäß Anspruch 1 oder 2, wobei der Ton vom Hectorit-Typ ein synthetischer Ton ist.

4. Verfahren gemäß einem der vorstehenden Ansprüche, bei dem die hergestellte Zubereitung einen pH unterhalb von etwa 8,5 hat.

5. Verfahren gemäß einem der vorstehenden Ansprüche, umfassend die Zugabe von Cellulosegummi als Verdickungsmittel.

6. Verfahren gemäß Anspruch 5, wobei das Cellulosegummi Carboxymethylcellulosegummi ist.

7. Verfahren, die einen Ton vom Hectorit-Typ und Cellulosegummi in ausreichender Menge, um eine synergistische Erhöhung der Viskosität zu zeigen, sowie ein Konservierungsmittel enthält, wobei die Zubereitung im wesentlichen frei von oberflächenaktiven und die Augen reizenden Mitteln ist.

8. Verfahren zum Herstellen einer nichtöligen Zubereitung zum Reinigen der Haut, das im wesentlichen das Vereinigen von Ton vom Hectorit-Typ in einer Menge von bis zu etwa 2 Gew.-%, einem Konservierungsmittel und ggf. einem Verdickungsmittel, einem Farbstoff und einem Feuchthaltemittel umfaßt, wobei die Zubereitung im wesentlichen frei von sowohl oberflächenaktiven als auch die Augen reizenden Mitteln ist.

9. Verfahren gemäß Anspruch 7 oder 8, in der der Ton vom Hectorit-Typ und/oder das Cellulosegummi gemäß einem der Ansprüche 1 bis 6 enthalten sind.

10. Verfahren zum Reinigen der im Bereich der Augen befindlichen, empfindlichen Haut durch Auftragen einer nichtöligen Zubereitung, die eine die Haut reinigende Menge Ton vom Hectorit-Typ bis zu etwa 2 Gew.-% enthält, wobei die Zubereitung im wesentlichen frei von oberflächenaktiven und die Augen reizenden Mitteln ist.

11. Verfahren gemäß Anspruch 10, bei dem die Zubereitung gemäß einem der Ansprüche 1 bis 9 definiert ist.

12. Verwendung von Ton vom Hectorit-Typ in einer Menge von bis zu etwa 2 Gew.-% in einer Zubereitung zum Reinigen der Haut, die im wesentlichen frei von oberflächenaktiven und die Augen reizenden Mitteln ist, zum Unterstützen der Entfernung von Verunreinigungen von empfindlicher Haut oder Haaren.

13. Zubereitung, die gemäß einem der Ansprüche 1 bis 9 hergestellt werden kann.

## Revendications (Revendications pour l'(les) Etat(s) contractant(s) suivant(s): AT, BE, CH, LI, DE, DK, FR, GB, GR, IE, IT, LU, MC, NL, SE)

1. Composition non huileuse de démaquillage, comprenant une quantité démaquillante d'une argile du type hectorite représentant jusqu'à environ 2 % en poids de la composition et un conservateur, ladite composition étant pratiquement dépourvue de surfactants et d'irritants oculaires.

2. Composition suivant la revendication 1, dans laquelle l'argile du type hectorite est présente en une quantité d'environ 0,25 % à 1,8 % en poids.

3. Composition suivant la revendication 1 ou la revendication 2, dans laquelle l'argile du type hectorite est une argile synthétique.

4. Composition suivant l'une quelconque des revendications précédentes, ayant un pH inférieur à environ 8,5.

5. Composition suivant l'une quelconque des revendications précédentes, comprenant une gomme de cellulose comme épaississant.

6. Composition suivant la revendication 5, dans laquelle la gomme de cellulose est une gomme de carboxyméthylcellulose.

7. Composition comprenant une argile du type hectorite et une gomme de cellulose en des quantités suffisantes pour provoquer une augmentation synergique de viscosité, et un conservateur, ladite composition étant pratiquement dépourvue de surfactants et d'irritants oculaires.

8. Composition non huileuse de démaquillage, consistant essentiellement en une argile du type hectorite en une quantité allant jusqu'à environ 2 % en poids et un conservateur ainsi que, facultativement, un épaississant, un agent colorant et un agent d'humectation, et qui est pratiquement dépourvue de surfactants et d'irritants oculaires.

9. Composition suivant la revendication 7 ou la revendication 8, dans laquelle l'argile du type hectorite et/ou la gomme de cellulose sont incorporées de la manière définie dans l'une quelconque des revendications 1 à 6.

10. Procédé de démaquillage de la peau sensible dans la région des yeux par application d'une composition non huileuse comprenant une quantité démaquillante d'une argile du type hectorite allant jusqu'à environ 2 % en poids, ladite composition étant pratiquement dépourvue de surfactants et d'irritants oculaires.

11. Procédé suivant la revendication 10, dans lequel la composition répond à la définition suivant l'une quelconque des revendications 1 à 9.

12. Utilisation d'une argile du type hectorite en une quantité allant jusqu'à environ 2 % en poids dans une composition de démaquillage pratiquement dépourvue de surfactants et d'irritants oculaires pour faciliter l'élimination des impuretés de la peau sensible ou des cheveux.

13. Procédé de préparation d'une composition suivant l'une quelconque des revendications 1 à 9, par association d'une argile du type hectorite avec un conservateur et un solvant convenable.

## Revendications (Revendications pour l'(les) Etat(s) contractant(s) suivant(s): ES)

1. Procédé de préparation d'une composition non huileuse de démaquillage, comprenant l'association d'une quantité démaquillante d'une argile du type hectorite allant jusqu'à environ 2 % en poids de la composition avec de l'eau et un conservateur, ladite composition étant pratiquement dépourvue de surfactants et d'irritants oculaires.

2. Procédé suivant la revendication 1, dans lequel l'argile du type hectorite est présente en une quantité d'environ 0,25 % à 1,8 % en poids.

3. Procédé suivant la revendication 1 ou la revendication 2, dans lequel l'argile du type hectorite est une argile synthétique.

4. Procédé suivant l'une quelconque des revendications précédentes, dans lequel la composition préparée a un pH inférieur à environ 8,5.

5. Procédé suivant l'une quelconque des revendications précédentes, comprenant l'addition d'une gomme de cellulose comme épaississant.

6. Procédé suivant la revendication 5, dans lequel la gomme de cellulose est une gomme de carboxyméthylcellulose.

7. Procédé de préparation d'une composition, comprenant l'association d'une argile du type hectorite avec une gomme de cellulose, en des quantités suffisantes pour provoquer une augmentation synergique de viscosité, et un conservateur, ladite composition étant pratiquement dépourvue de surfactants et d'irritants oculaires.

8. Procédé de préparation d'une composition non huileuse de démaquillage, consistant essentiellement en l'association d'une argile du type hectorite en une quantité allant jusqu'à environ 2 % en poids avec un conservateur et, facultativement, un épaississant, un agent colorant et un agent d'humectation, ladite composition étant pratiquement dépourvue de surfactants et d'irritants oculaires.

9. Procédé suivant la revendication 7 ou la revendication 8, dans lequel l'argile du type hectorite et/ou la gomme de cellulose sont incorporées de la manière définie dans l'une quelconque des revendications 1 à 6.

10. Procédé de démaquillage de la peau sensible dans la région des yeux par application d'une composition non huileuse comprenant une quantité démaquillante d'une argile du type hectorite allant jusqu'à environ 2 % en poids, ladite composition étant pratiquement dépourvue de surfactants et d'irritants oculaires.

11. Procédé suivant la revendication 10, dans lequel la composition répond à la définition suivant l'une quelconque des revendications 1 à 9.

12. Utilisation d'une argile du type hectorite en une quantité allant jusqu'à environ 2 % en poids dans une composition de démaquillage pratiquement dépourvue de surfactants et d'irritants oculaires pour faciliter l'élimination des impuretés de la peau sensible ou des cheveux.

13. Composition qui peut être préparée par l'un quelconque des procédés suivant les revendications 1 à 9.
